# EUROPEAN PATENT APPLICATION

(11) **EP 4 173 586 A1**
(43) Date of publication of application: **03.05.2023**
(21) Application number: 21830248.7
(22) Date of filing: 23.06.2021
(51) Int. Cl.: A61B 34/30, A61B 17/00

(54) **LAPAROSCOPE BACTERIA-ISOLATION LOCKING CONNECTOR AND LAPAROSCOPIC SURGERY ROBOT**

(30) Priority: 24.06.2020 CN 202010589585
(71) Applicant: Harbin Intelligent Surgery Equipment Co., Ltd., Harbin, Heilongjiang 150000 (CN)
(72) Inventor: WANG, Jianguo, Harbin, Heilongjiang 150000 (CN); WANG, Xiaowei, Harbin, Heilongjiang 150000 (CN)
(74) Representative: Zaboliene, Reda
(86) International application number: PCT/CN2021/101726
(87) International publication number: WO 2021/259306

(57) **Abstract**

The present invention relates to the technical field of medical instruments. Provided are a laparoscope bacteria-isolation locking connector and a laparoscopic surgery robot. The laparoscope bacteria-isolation locking connector comprises: a laparoscope connector apparatus fixedly mounted into a mounting hole of a sliding table; a laparoscope locking knob, a lower end hole of the laparoscope locking knob adapted to be connected to the end portion of the laparoscope connector apparatus; a laparoscope connector upper cover adapted and connected to an upper end hole of the laparoscope locking knob; and a laparoscope locking apparatus mounted between the laparoscope connector apparatus and the laparoscope connector upper cover, located in a middle hole of the laparoscope locking knob, and used for implementing locking and opening of a laparoscope. According to the present invention, the sterile laparoscope is fixed to the sliding table on an operation arm by means of the bacteria-isolation locking connector, so that the laparoscope is always in a sterile state when moving following the operation arm of the robot.

## Description

### Technical Field

The present invention relates to a medical instrument, and more particularly, to a laparoscope bacteria-isolation locking connector and a laparoscopic surgery robot.

### Background Art

Laparoscopic surgery is a newly developed minimally invasive method, which is an inevitable trend of surgical methods in the future. With the integration of related sciences, it has laid a strong foundation for the development of new technologies and methods, and more and more proficient doctors have made many past open surgeries replaced by endovascular surgeries, greatly increasing the chance of surgical options.

For the traditional laparoscopic surgeries, a doctor's assistant holds a laparoscope, and the doctor giving the surgery holds a surgical instrument to perform an endoluminal surgical operation, which requires the cooperation of two or even more people. Operation is easy to be restricted, the field of view of the mirror is unstable, and the patient is easy to be infected. Nowadays, laparoscopic surgery robots are gradually replacing the operation mode of traditional laparoscopic surgery, and are being accepted by more and more doctors and patients. However, if it is not ensured that the laparoscope is in a sterile state after being fixed on operation arms of a surgery robot, infection and other consequences may be caused.

### Summary of the Invention

In order to overcome the above-mentioned problems, the present invention provides the following technical solutions:
a laparoscope bacteria-isolation locking connector for use on a laparoscopic surgery robot, which comprises
a laparoscope connector apparatus adapted to be fixedly provided within a mounting hole of a sliding table of the laparoscopic surgery robot;
a laparoscope locking knob sleeved on the laparoscope connector apparatus;
a laparoscope connector upper cover snap-fitted on an upper end surface of the laparoscope locking knob;
a laparoscope locking apparatus located inside the laparoscope locking knob, and upper and lower ends of the laparoscope locking apparatus are respectively connected to the laparoscope connector upper cover and the laparoscope connector apparatus, and the laparoscope locking apparatus achieves locking and opening of a laparoscope by rotating the laparoscope locking knob;
the laparoscope connector apparatus, the laparoscope locking apparatus and the laparoscope connector upper cover together form an inner space for accommodating the laparoscope.

Alternatively, the laparoscope connector apparatus comprises a laparoscope upper connector structure and a laparoscope lower connector structure, wherein the laparoscope upper connector structure comprises a fan-shaped boss, an upper cylindrical tube and a lower cylindrical tube connected successively, the outside of the fan-shaped boss is sleeved with the laparoscope locking knob, and the lower cylindrical tube is connected with the laparoscope lower connector structure.

Alternatively, the number of the fan-shaped bosses is two, and two of the fan-shaped bosses are symmetrically provided at the top of the upper cylindrical tube.

Alternatively, two protruding cylinders are symmetrically provided on the outer circumference of the lower cylindrical tube, the laparoscopic lower connector structure is provided with a sliding groove matching the protruding cylinders, and when the laparoscopic upper connector structure rotates relative to the laparoscopic lower connector structure, the outer wall surface of the lower cylindrical tube is adapted to be connected to the inner wall surface of the laparoscopic lower connector structure.

Alternatively, the sliding groove comprises an inclined region and a horizontal region, the beginning of the inclined region is connected to a circular arc transition of the upper end surface of the laparoscopic lower connector structure, and an end portion of the inclined region is in circular arc transition connection with the starting end of the horizontal region.

Alternatively, the laparoscope locking apparatus comprises a laparoscope locking buckle and locking buckle spindles provided through the laparoscope locking buckle, the two locking buckle spindles and the two fan-shaped bosses are provided at intervals, and the two laparoscope locking buckles and the two locking buckle spindles are symmetrically provided around the central axis of the laparoscope connector apparatus;
one end of the locking buckle spindle is inserted into a first end face hole of the upper connector structure of the laparoscope, and the other end of the locking buckle spindle is inserted into a second end face hole of the laparoscope connector upper cover.

Alternatively, the laparoscopic locking buckle comprises an interconnected locking buckle body and a first connection portion, the locking buckle body is located on a side proximal to the laparoscope locking knob and the first connection portion located on a side away from the laparoscope locking knob.

Alternatively, the laparoscope locking buckle further comprises a first protruding portion provided at an end portion of the fastener body, the first protruding portion is adjacent to an inner wall of the laparoscope locking knob, the inner wall of the laparoscope locking knob is symmetrically provided with a second boss, and the second protruding portion abuts against the first protruding portion during rotational locking of the laparoscope locking knob.

Alternatively, two semi-cylindrical protrusions are symmetrically provided on the outer circumference of the upper cylindrical tube, the semi-cylindrical protrusions are adapted to be connected with the semi-cylindrical clamping grooves of the inner wall of the sliding table, and the inside of the laparoscope connector apparatus is isolated from the sliding table to form a sterile space.

The present invention provides a laparoscope bacteria-isolation locking connector which is advantageous compared with the prior art in that: the sterile laparoscope is fixed on the sliding table of the operation arm of the surgery robot through the bacterial isolation locking interface to realize the follow-up between the laparoscope and the operation arm of the robot. In addition, the bacterial isolation locking connector of the laparoscope makes the laparoscope always in a sterile state and easy to operate, which not only ensures the stable view of the laparoscope, but also ensures that the patient is not infected.

The present invention also provides a laparoscopic surgery robot comprising a sliding table and the laparoscope bacteria-isolation locking connector described above, and the laparoscope bacteria-isolation locking connector is fixedly connected to the sliding table.

The advantages of the laparoscopic surgery robot according to the present invention and the laparoscope bacteria-isolation locking connector with respect to the prior art are the same and will not be described in detail herein.

### Brief Description of the Drawings

FIG. 1 is a schematic view illustrating a structure of a laparoscope bacteria-isolation locking connector according to an embodiment of the present invention;
FIG. 2 is a top view of FIG. 1 with a laparoscope connector upper cover open;
FIG. 3 is a front view of a laparoscope bacteria-isolation locking connector according to an embodiment of the present invention;
FIG. 4 is a cross-sectional view taken along the direction A-A after the laparoscope upper connector structure, the laparoscope locking apparatus, the laparoscope locking knob and the laparoscope connector upper cover are installed in an embodiment of the present invention;
FIG. 5 is a partially enlarged schematic view of a portion C of FIG. 4;
FIG. 6 is a cross-sectional view taken along the direction B-B after the laparoscope upper connector structure, the laparoscope locking apparatus, the laparoscope locking knob and the laparoscope connector upper cover are installed in an embodiment of the present invention;
FIG. 7 is a schematic view illustrating a state of an installation process of a laparoscope bacteria-isolation locking connector according to an embodiment of the present invention;
FIG. 8 is a schematic view illustrating a locking state of a laparoscope according to an embodiment of the present invention;
FIG. 9 is a top view of FIG. 8 with a laparoscope connector upper cover open;
FIG. 10 is a schematic view of an open state of a laparoscope according to an embodiment of the present invention;
FIG. 11 is a top view of FIG. 10 with a laparoscope connector upper cover open;
FIG. 12 is a schematic view illustrating a state in which a laparoscope bacteria-isolation locking connector is installed according to an embodiment of the present invention.

### Description of Reference Numerals:

1-laparoscope connector apparatus; 11-laparoscope upper connector structure; 111-upper cylindrical tube; 112-lower cylindrical tube; 1121-protruding cylinder; 113-semi-cylindrical protrusion; 114-fan-shaped boss; 12-laparoscopic lower connector structure; 121-sliding groove; 1211-inclined region; 1212-horizontal region; 13-first end face hole; 2-laparoscope locking knob; 21-lower end hole; 22-upper end hole; 23-middle hole; 24-second protruding portion; 3-laparoscope connector upper cover; 31-second end face hole; 4-laparoscope locking apparatus; 41-laparoscope locking buckle; 411-locking buckle body; 412-first connection portion; 413-first protruding portion; 42-locking buckle spindle; 5-sliding table; 51-mounting hole; 511-semi-cylindrical clamping groove.

### Detailed Description of the Invention

The above and other technical features and advantages of the present invention will be described in more detail with reference to the accompanying drawings.

In addition, in the description of the present invention, it should be understood that the forward direction of "X" in the drawings represents the left and, correspondingly, the reverse direction of "X" represents the right; the forward direction of "Y" represents the front and, correspondingly, the reverse direction of "Y" represents the rear; the forward direction of "Z" represents upward and, correspondingly, the reverse direction of "Z" represents downward. The orientation or positional relationships indicated by the terms "X", "Y", "Z", etc. are merely for convenience in describing and simplifying the present invention and do not indicate or imply that the device or element being referred to must have a particular orientation, be constructed and operated in a particular orientation and, therefore, should not be construed as limiting the invention. If there is a description of "first", "second", etc. in an embodiment of the invention, the description of "first", "second", etc. is for descriptive purposes only and is not to be construed as indicating or implying a relative importance or implicitly indicating the number of technical features indicated.

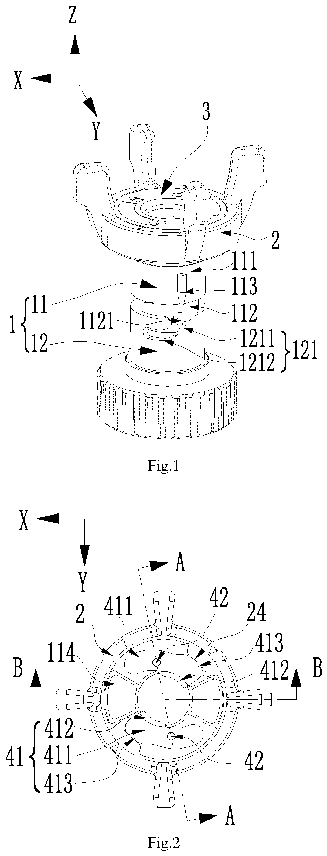

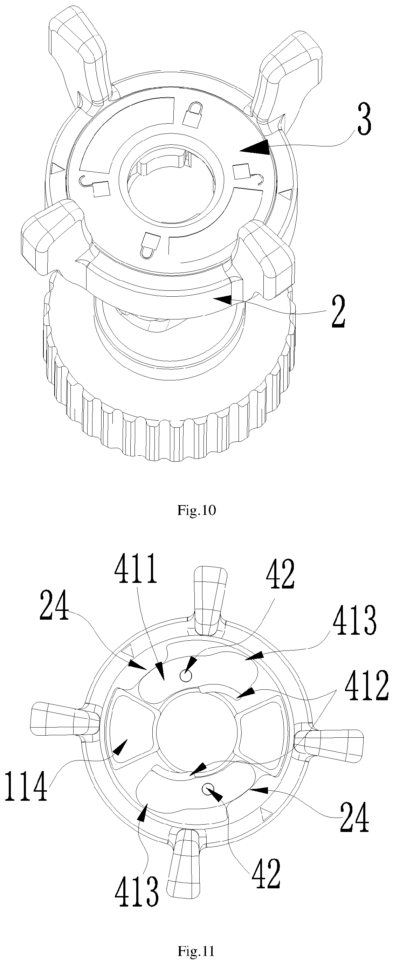

As shown in FIGS. 1-12, an embodiment of the present invention provides a laparoscope bacteria-isolation locking connector for a laparoscopic surgery robot, which comprises a laparoscope connector apparatus 1, a laparoscope locking knob 2, a laparoscope connector upper cover 3 and a laparoscope locking apparatus 4, wherein the laparoscope connector apparatus 1 is fixedly mounted in a mounting hole 51 of a sliding table 5, the laparoscope connector apparatus 1, the laparoscope connector upper cover 3 and the laparoscope locking apparatus 4 together form an inner space for accommodating a laparoscope, and the laparoscope locking knob 2 is sleeved on the laparoscope connector apparatus 1. A laparoscope connector upper cover 3 is snap-fitted on an upper end surface of the laparoscope locking knob 2, a laparoscope locking apparatus 4 is located inside the laparoscope locking knob 2, and the laparoscope locking apparatus 4 is installed between the laparoscope connector apparatus 1 and the laparoscope connector upper cover 3; upper and lower ends of the laparoscope locking apparatus 4 are respectively connected to the laparoscope connector upper cover 3 and the laparoscope connector apparatus 1; after the laparoscope locking knob 2 is installed, an upper end portion of the laparoscope connector apparatus 1 and the laparoscope connector upper cover 3 are welded together; and the laparoscope locking apparatus 4 is used for locking and opening a laparoscope.

In this embodiment, the internal space formed by the laparoscope connector apparatus 1, the laparoscope connector upper cover 3 and the laparoscope locking apparatus 4 together is used for accommodating the laparoscope, so that the internal accommodating space is isolated from the outside; the laparoscope is inserted into the interior of the laparoscope connector apparatus 1, and the contact surfaces thereof are all in a sterile state, which meets the requirements for aseptic use of the laparoscope. Since the laparoscope locking apparatus 4 can lock and open the laparoscope under the rotary drive of the laparoscope locking knob 2, the structure design is clever and the operation is convenient.

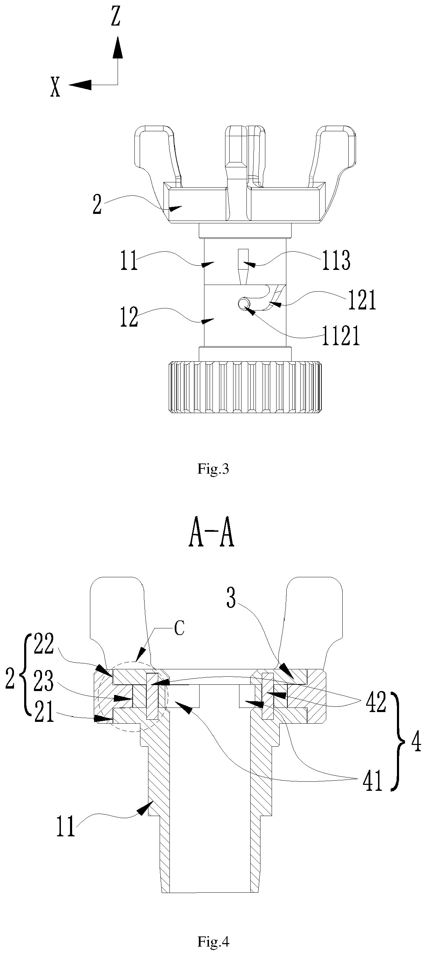

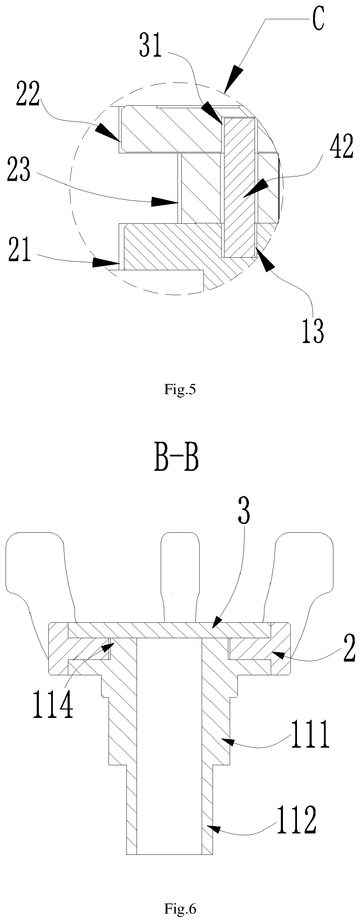

Specifically, the laparoscope locking knob 2 is made of hard plastic, and a plurality of handles are uniformly provided with an upward extension of an outer circumferential portion of the laparoscope locking knob 2, which is mainly used as an action site when the laparoscope locking knob 2 is rotated to apply an external force to facilitate operation. In this embodiment, four handles are preferably provided, and the four handles are uniformly and symmetrically provided along the circumferential direction, with a simple structure and meeting the requirements for use. As shown in FIGS. 4 and 5, in this embodiment, the middle of the laparoscope locking knob 2 is further provided with an I-shaped through-hole, comprising a lower end hole 21, an upper end hole 22 and a middle hole 23, wherein the lower end hole 21 is adapted to be connected to the end portion of the laparoscope upper connector structure 11, the middle hole 23 is used for accommodating the laparoscope locking apparatus 4, and the upper end hole 22 is in a snap-fit connection with the laparoscope connector upper cover 3 to be isolated from a sterile space of the outside, so that the laparoscope locking apparatus 4 can be rotated in the middle hole 23 in order to achieve the locking and opening of the laparoscope in an internal sterile space. The upper end surface of the laparoscope locking knob 2 is also marked with an arrow to indicate the relevant icon when rotated.

Specifically, the laparoscope connector upper cover 3 is made of a hard plastic, and the lower surface thereof is connected to the end surface of the fan-shaped boss 114 of the upper end surface of the laparoscope upper connector structure 11 by ultrasonic welding, and the welding is relatively reliable, so that the inner space of the laparoscope upper connector structure 11 is isolated from the external bacteria space; of course, other sealing methods can also be used to satisfy the need of forming a sealing space and to be practical. The laparoscope connector upper cover 3 is further provided with two icons of "lock open" and "lock close" corresponding to the rotation region of the laparoscope locking knob 2. It corresponds to the arrow mark on the upper end surface of laparoscope locking knob 2 to indicate the locking and opening mark of laparoscope after rotation.

In some embodiments, the laparoscope connector apparatus 1 comprises a laparoscope upper connector structure 11 and a laparoscope lower connector structure 12, and as shown in FIGS. 1-6, the laparoscope upper connector structure 11 comprises a coaxial upper and upper cylindrical tube 111, a lower cylindrical tube 112 and a fan-shaped boss 114, two protruding cylinders 1121 are symmetrically provided on the outer circumference of the lower cylindrical tube 112, and the end portions of the protruding cylinders 1121 are rounded and angle-processed to facilitate hanging to enable the connection between the laparoscope upper connector structure 11 and the laparoscope lower connector structure 12. The upper end of the laparoscope lower connector structure 12 is correspondingly provided with a sliding groove 121 properly connected with the protruding cylinder 1121; at the same time, in order to connect the laparoscope upper connector structure 11 with the sliding table 5 at the robot arm, the sliding groove 121 is clamped with the protruding cylinder 1121 in the mounting hole 51 of the sliding table 5 by means of the rotation of the laparoscope lower connector structure 12, and in order to make the laparoscope upper connector structure 11 and the laparoscope lower connector structure 12 fit together, the outer wall of the lower cylindrical tube 112 is properly connected with the inner wall of the laparoscope lower connector structure 12.

In this embodiment, under the rotation of the laparoscope lower connector structure 12, the sliding groove 121 is driven to rotate, so that the sliding groove 121 and the protruding cylinder 1121 are rotatably hitched, and at the same time, the lower cylindrical tube 112 fits against the inner wall of the laparoscope lower connector structure 12 and is inserted, so that the outer wall of the lower cylindrical tube 112 abuts against the inner wall of the laparoscope lower connector structure 12, but can rotate with each other to achieve the fit connection between the laparoscope upper connector structure 11 and the laparoscope lower connector structure 12, which is simple in operation and reliable in sealing. Furthermore, it can ensure that the laparoscope connector apparatus 1 cannot communicate with the outside; in addition, the laparoscope connector apparatus 1 as a whole is used as a sterile product; after the upper laparoscope connector structure 11 and the lower laparoscope connector structure 12 are attached and connected, the inner surfaces thereof are in a sterile state to ensure an inner sterile space; and the sliding table 5 on the mechanical arm is in a sterile state, when the laparoscope connector apparatus 1 is loaded into the sliding table 5 and then the laparoscope set is fixed inside the laparoscope connector apparatus 1, the laparoscope can always be in contact with the sterile surface inside the laparoscope connector apparatus 1, therefore, the laparoscope is always sterile and meets the actual use requirements.

In some embodiments, the end face of the laparoscope upper connector structure 11 is further provided with a fan-shaped boss 114, two fan-shaped bosses 114 are symmetrically provided along a central axis of the laparoscope connector apparatus 1 and are located in the middle hole 23 of the laparoscope locking knob 2, and the fan-shaped bosses 114 are provided at intervals from the laparoscope locking apparatus 4, on the one hand, for realizing the limiting and blocking of the laparoscope locking knob 2, and on the other hand, for satisfying the connection requirements between the upper end surface of the fan-shaped bosses 114 and the laparoscope connector upper cover 3.

Specifically, the laparoscope locking apparatus 4 comprises laparoscope locking buckles 41 and locking buckle spindles 42, wherein the locking buckle spindle 42 penetrates the laparoscope locking buckle 41, two laparoscope locking buckles 41 and two locking buckle spindles 42 are symmetrically provided around the central axis of the laparoscope connector apparatus 1, one end of the locking buckle spindle 42 is inserted into the first end face hole 13 of the laparoscope upper connector structure 11, and the other end of the locking buckle spindle 42 is inserted into the second end face hole 31 of the laparoscope connector upper cover 3; laparoscopic locking buckles 41 are connected between the upper end surface of the laparoscope connector apparatus 1 and the lower end surface of the laparoscopic connector upper cover 3 by means of corresponding locking buckle spindles 42.

In some embodiments, as shown in FIG. 2, it is preferred that the laparoscope locking apparatus 4 uses two laparoscope locking buckles 41 rotating around corresponding locking buckle spindles 42, the two laparoscope locking buckles 41 and the two locking buckle spindles 42 are symmetrically provided around the central axis of the laparoscope connector apparatus 1, and the two laparoscope locking buckles 41 are oppositely provided around the central axis of the laparoscope connector apparatus 1 to achieve the locking and unlocking of laparoscopes with reliable connection. Of course, in some other embodiments, the laparoscope locking apparatus 4 may have other configurations, such as springs, gears, etc. to facilitate handling, simple construction, and to enable laparoscopic locking.

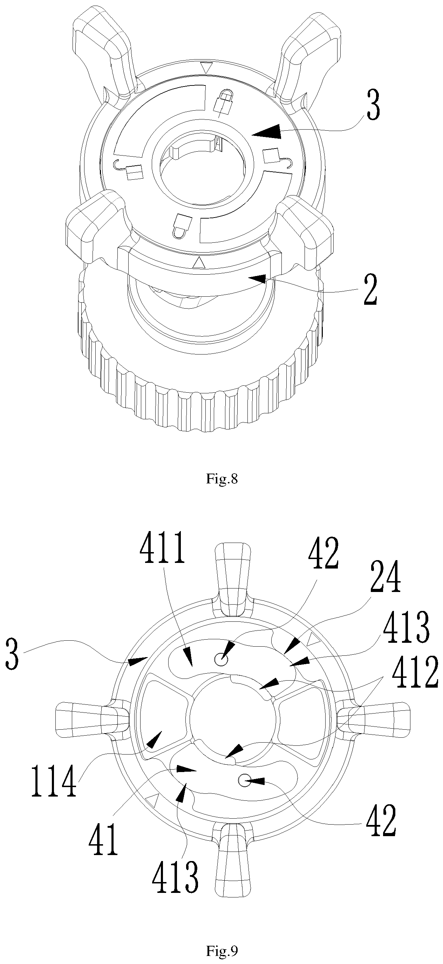

Preferably, in this embodiment, as shown in FIGS. 8-11, the laparoscope locking buckle 41 includes a locking buckle body 411 and a first connection portion 412 located at an end portion of opposite sides of the two locking buckle bodies 411, the first connection portion 412 is made of soft rubber, and the two first connection portions 412 are symmetrically provided along the central axis of the laparoscope connector apparatus 1.

The inner wall of the locking buckle body 411 away from the laparoscope locking knob 2 is partially embedded with a first connection portion 412 made of soft rubber, and the laparoscope locking buckles 41 are used for locking or releasing the laparoscope. Specifically, the whole laparoscope bacteria-isolation locking connector comprises a laparoscope connector apparatus 1, a laparoscope locking knob 2, a laparoscope connector upper cover 3 and a laparoscope locking apparatus 4, wherein the laparoscope connector apparatus 1 and the laparoscope connector upper cover 3 are made of hard plastic, have a high structural strength, are not easily damaged and are connected reliably, and at the same time, the laparoscope connector upper cover 3 can be in sealed connected with the upper end surface of the laparoscope connector apparatus 1 by means of ultrasonic welding; compared with other welding methods, the ultrasonic welding speed is very fast and does not generate spark, and the welding material does not melt, so that the sealed connection achieved by the welding method is more reliable. The laparoscope locking knob 2 and the laparoscope locking apparatus 4 can be made of plastic; the laparoscope locking buckles 41 are made of hard plastic parts; the internal wall of the laparoscope locking buckle 41 away from the laparoscope locking knob 2 is embedded with a first connection portion 412 in the contact part with the laparoscope; the first connection portion 412 is made of soft rubber to facilitate the locking of the laparoscope and prevent slipping; and the material is relatively light, and relatively more simple and labor-saving in operation. In this embodiment, the material of the laparoscope locking knob 2 and the laparoscope locking apparatus 4 is not limited to meet the functional requirements and achieve a compact structure, sensitive response, long life and reliable use.

Preferably, the laparoscope locking buckle 41 further comprises a first protruding portion 413 provided at the end portion of the locking buckle body 411, the first protruding portion 413 is provided close to the inner wall of the laparoscope locking knob 2, the inner wall of the laparoscope locking knob 2 is symmetrically provided with a second protruding portion 24, and during the rotation locking of the laparoscope locking knob 2, the second protruding portion 24 abuts against the first protruding portion 413.

In this embodiment, as shown in FIGS. 8-11, the inner side surface of the laparoscope locking knob 2 is integrally formed with a second protruding portion 24, two second protruding portions 24 are symmetrically provided, and the other side of the locking buckle body 411 away from the first connection portion 412 is provided with a first protruding portion 413, when the laparoscope locking knob 2 is manually rotated to one side, the second protruding portion 24 is driven to move inside, and when the second protruding portion 24 moves into contact with the first protruding portion 413, the laparoscope locking buckles 41 rotate around the laparoscope locking buckle spindles 42 under the action of the external force of the second protruding portion 24. And moving the two first connection portions 412 towards the intermediate shaft, and when moving until the second protruding portion 24 abuts against the fan-shaped boss 114 on the end face of the upper and upper cylindrical tubes 111, the second protruding portion is locked and rotated into position to lock the laparoscope; when the laparoscope locking knob 2 is manually rotated in the other side direction, the second protruding portion 24 is separated from the first protruding portion 413, the second protruding portion 24 drives the laparoscope locking buckles 41 to return to the original position around the laparoscope locking buckle spindles 42 during the movement, and when the second protruding portion 24 abuts against the fan-shaped boss 114 on the upper end surface of the laparoscope connector apparatus 1 on the other side, the second protruding portion 24 is opened and rotated into position to achieve the opening of the laparoscope, with a simple structure and ingenious design.

The second protruding portion 24 inside the laparoscope locking knob 2 is in contact with the laparoscope locking buckle 41 during rotation, and when the first protruding portion 413 of the laparoscope locking buckle 41 approaches the inner side surface of the laparoscope locking knob 2, the laparoscope locking buckle 41 rotates outwards around the laparoscope locking buckle spindle 42, and when the arrow mark on the laparoscope locking knob 2 points to the "lock open" icon on the laparoscope connector upper cover 3, the contact surface between the first connection portion 412 and the laparoscope is opened; when the laparoscope is gently inserted into the proper position, the laparoscope locking knob 2 in the opposite direction is rotated, so that the arrow mark on the laparoscope locking knob 2 points to the "lock close" icon on the laparoscope connector upper cover 3. At this time, the second protruding part 24 of the laparoscope locking knob 2 presses the first protruding part 413 of the laparoscope locking buckle 41, so that the laparoscope locking buckle 41 rotates inwards, thereby clamping the contact surface with the laparoscope, i.e., the laparoscope is locked.

In some embodiments, the upper cylindrical tubes 111 of the laparoscope upper connector structure 11 and the laparoscope lower connector structure 12 have the same tube diameter to facilitate the fitting connection of the two together at the end face and make the assembly more compact, and at the same time, in order to enable the laparoscope lower connector structure 12 to be smoothly inserted into the mounting hole 51 of the sliding table 5, the tube diameter is less than or equal to the diameter of the sliding table mounting hole 51 to facilitate the mounting operation.

It can be understood that the connection between the laparoscope upper connector structure 11 and the laparoscope lower connector structure 12 may also adopt other connection methods besides the rotary hanging connection, such as a pin connection, a screw connection, etc. and the purpose thereof is to achieve a fixed connection between the laparoscope upper connector structure 11 and the laparoscope lower connector structure 12 to form a space.

In some embodiments, the sliding groove 121 comprises an inclined region 1211 and a horizontal region 1212, wherein the starting end of the inclined region 1211 is in arc transition connection with the upper end surface of the laparoscope lower connector structure 12, and the end portion of the inclined region 1211 is in arc transition connection with the starting end of the horizontal region 1212, and the end portion of the horizontal region 1212 is of a horizontal semi-circular hole structure.

In this embodiment, as shown in FIGS. 1 and 3, the upper right side of the sliding groove 121 is the starting end of the inclined region 1211, both sides of the starting end are in smooth transition connection with the end surface of the lower connector structure 12 of the laparoscope, the middle is the intersection region of the starting end of the inclined region 1211 and the horizontal region 1212, both the upper and lower sides of the intersection are subjected to an arc transition treatment, the lower left side of the sliding groove 121 is the end portion of the horizontal region 1212, and a semi-circular hole transition treatment is performed, and at the same time, the difference between the minimum hole of the starting end of the inclined region 1211 and the diameter of the protruding cylinder 1121 is d1, and the value range of d1 is from -0.1 to 0mm. The difference between the diameter of the semi-circular hole at the end portion of the horizontal region 1212 and the diameter of the protruding cylinder 1121 is d2, and the value range of d2 is from 0 to 0.2mm; and the above-mentioned arrangement of each connection portion ensures that the clamping can be smooth during the whole process of the rotary clamping.

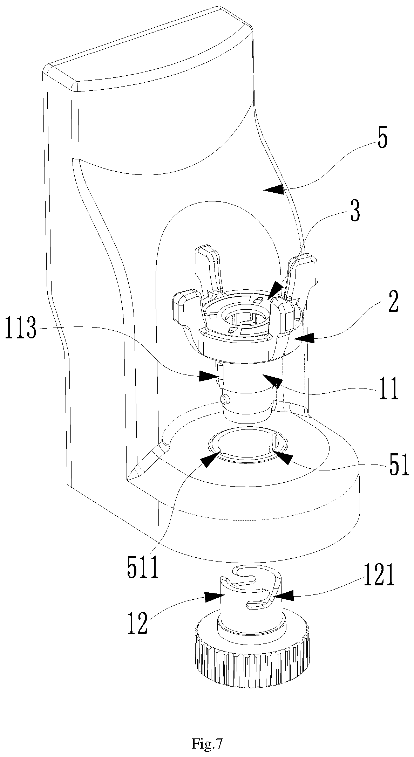

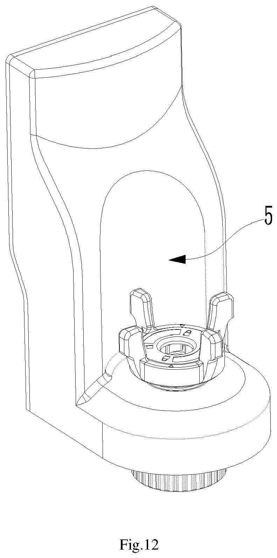

In this embodiment, as shown in FIGS. 7 and 12, since the upper connector structure 11 of the laparoscope is connected to the lower connector structure 12 of the laparoscope by means of a rotary hanging connection, and under the condition of line of sight being blocked, in order to facilitate a more reliable rotary hanging connection during manual operation, a smooth transition connection is provided between the inclined region 1211 and the horizontal region 1212 of the sliding groove 121; the minimum hole of the starting end of the inclined region 1211 of the sliding groove 121 is slightly smaller than the diameter of the protruding cylinder 1121, that is to say, there is an interference fit between the starting end of the inclined region 1211 and the protruding cylinder 1121, with the interference amount being 0.1mm at the maximum; the end portion of the slide in the horizontal area 1212 of the slide groove 121 is provided in a horizontal semi-circular shape, and the size of the semi-circular hole is slightly greater than the diameter of the protruding cylinder 1121, that is, the end portion of the slide groove and the protruding cylinder 1121 are in a clearance fit, with the maximum clearance being 0.2mm, and the benefit of this design is that when rotating and hanging, the fingers have an obvious sense of layers, so that there is a strong hand feeling of a locked state, and same cannot easily slide out after being locked.

In some embodiments, the end face distance of the two protruding cylinders 1121 is less than the outer diameter of the laparoscopic lower connector structure 12.

In this embodiment, the distance between the end surfaces of the protruding cylinder 1121 is specifically limited so that the laparoscopic lower connector structure 12 can be inserted from below the mounting hole 51 of the sliding table 5 and can be rotatably mounted in the mounting hole 51 as long as the above-mentioned connection relationship is satisfied.

Preferably, in this embodiment, as shown in FIGS. 1 and 3, an upper cylindrical tube 111 is provided on the laparoscope upper connector structure 11, two semi-cylindrical protrusions 113 are symmetrically provided on the outer circumference of the upper cylindrical tube 111, a corresponding semi-cylindrical clamping groove 511 is provided on an inner wall of the mounting hole 51 of the sliding table 5 in a vertical direction, and during mounting, the two semi-cylindrical protrusions 113 are vertically inserted into the semi-cylindrical clamping groove 511 on the inner wall of the sliding table 5 to limit the laparoscope upper connector structure 11, which is reliable in connection and simple in operation.

Working principle: when a patient needs a surgery, the laparoscope bacteria-isolation locking connector of the present invention firstly inserts a stamp card into his body, swings a mechanical arm to an appropriate position, and a sliding table 5 is provided at the end portion of the mechanical arm; a sterile laparoscope upper connector structure 11 is inserted into a mounting hole 51 of the sliding table 5 from above the sliding table 5; two semi-cylindrical protrusions 113 of the laparoscope upper connector structure 11 are fixedly connected to semi-cylindrical clamping grooves 511 on the inner wall of the mounting hole 51; a laparoscope lower connector structure 12 is inserted into the laparoscope lower connector structure 12 from below the sliding table 5; and the laparoscope lower connector structure 12 is rotated, making the sliding groove 121 of the laparoscope lower connector structure 12 and the two protruding cylinders 1121 of the laparoscope upper connector structure 11 hitched inside the sliding table 5, rotating the laparoscope locking knob 2 in a side direction, so that the arrow mark thereon points to the "lock open" icon on the laparoscope connector upper cover 3; at this moment, the second protruding portion 24 of the laparoscope locking knob 2 is away from the first protruding portion 413 at one end of the laparoscope locking buckle 41, and during the rotation of the second protruding portion 24, the other end of the laparoscope locking buckle 41 is pressed, so that the laparoscope locking buckle 41 rotates outwards around the locking buckle spindle 42; thus, the contact surface between the first connection portion 412 and the laparoscope is opened, the laparoscope is gently inserted into an appropriate position, and the laparoscope locking knob 2 is rotated so that the arrow mark thereon points to the "lock close" icon on the upper cover 3 of the laparoscope interface, at the moment, the second protruding part 24 of the laparoscope locking knob 2 presses the first protruding part 413 at the other end of the laparoscope locking buckle 41, so that the laparoscope locking buckle 41 rotates inwards around the locking buckle spindle 42, thereby clamping the first connection portion 412 with the contact surface of the laparoscope, i.e., the laparoscope locking.

When the surgery is completed or the laparoscope needs to be replaced in the middle, the laparoscope locking knob 2 is rotated so that the arrow mark thereon points to the "lock open" icon on the laparoscope connector upper cover 3; at the moment, the laparoscope can be freely disassembled and rotated; since the laparoscope connector as a whole is a sterile product, and the sliding table 5 on the mechanical arm is in a sterile state, when the laparoscope is provided and fixed, the laparoscope can always be kept in contact with the sterile surface, so that the laparoscope is always in a sterile state, which meets the requirements for practical use.

Embodiments of the present invention also provide a laparoscopic surgery robot comprising a mechanical arm, a sliding table 5 and a laparoscope bacteria-isolation locking connector, wherein the laparoscope bacteria-isolation locking connector is fixedly connected to the sliding table 5 which is connected to the mechanical arm.

Therefore, an embodiment of the present invention provides a laparoscopic surgery robot, which can realize the relative rotation of the mechanical arm and the upright post, i.e. the mechanical arm can swing up and down relative to the upright post, and has a compact structure to satisfy the requirements of the degree of freedom when the laparoscopic surgery robot works; at the same time, a laparoscope bacteria-isolation locking connector is mounted on the sliding table 5 of the mechanical arm, and a sterile laparoscope is fixed on the operation arm of the surgery robot via the bacteria-isolating locking interface, so that the follow-up between the laparoscope and the operation arm of the robot can be realized; and the laparoscope bacteria-isolation locking connector enables the laparoscope to always be in a sterile state, which is easy to operate, and not only the field of view of the laparoscope is stable, but it can ensure that the patient is not infected.

Although the present disclosure has been described above, the scope of protection of the present disclosure is not limited thereto. Various changes and modifications may be affected by a person skilled in the art without departing from the spirit and scope of the disclosure, and it is intended that such changes and modifications fall within the scope of the appended claims.

## Claims

1. A laparoscope bacteria-isolation locking connector for use on a laparoscopic surgery robot, comprising a laparoscope connector apparatus (1) adapted to be fixedly provided in a mounting hole of a sliding table (5) of the laparoscopic surgery robot;
a laparoscope locking knob (2) sleeved on the laparoscope connector apparatus (1);
a laparoscope connector upper cover (3) snap-fitted on an upper end surface of the laparoscope locking knob (2); and
a laparoscope locking apparatus (4) located inside the laparoscope locking knob (2), wherein upper and lower ends of the laparoscope locking apparatus (4) are respectively connected to the laparoscope connector upper cover (3) and the laparoscope connector apparatus (1), and the laparoscope locking apparatus (4) achieves locking and opening of a laparoscope by rotating the laparoscope locking knob (2); wherein the laparoscope connector apparatus (1), the laparoscope locking apparatus (4) and the laparoscope connector upper cover (3) together form an inner space for accommodating the laparoscope.

2. The laparoscope bacteria-isolation locking connector according to claim 1, wherein the laparoscope connector apparatus (1) comprises a laparoscopic upper connector structure (11) and a laparoscopic lower connector structure (12), wherein the laparoscopic upper connector structure (11) comprises a fan-shaped boss (114), an upper cylindrical tube (111) and a lower cylindrical tube (112) which are connected successively, wherein the outside of the fan-shaped boss (114) is sleeved with the laparoscope locking knob (2), and the lower cylindrical tube (112) is connected to the laparoscopic lower connector structure (12).

3. The laparoscope bacteria-isolation locking connector according to claim 2, wherein the number of the fan-shaped bosses (114) is two, and two of the fan-shaped bosses (114) are symmetrically provided at the top of the upper cylindrical tube (111).

4. The laparoscope bacteria-isolation locking connector according to claim 2, wherein two protruding cylinders (1121) are symmetrically provided on the outer circumference of the lower cylindrical tube (112), a sliding groove (121) matching the protruding cylinders (1121) is provided on the laparoscopic lower connector structure (12), the protruding cylinders (1121) are adapted to snap-fit the sliding groove (121) when the laparoscopic upper connector structure (11) rotates relative to the laparoscopic lower connector structure (12), and an outer wall surface of the lower cylindrical tube (112) is adapted to connect with an inner wall surface of the laparoscopic lower connector structure (12).

5. The laparoscope bacteria-isolation locking connector according to claim 4, wherein the sliding groove (121) comprises an inclined region (1211) and a horizontal region (1212), a starting end of the inclined region (1211) is in arc transition connection with an upper end surface of the laparoscopic lower connector structure (12), and an end portion of the inclined region (1211) is in arc transition connection with a starting end of the horizontal region (1212).

6. The laparoscope bacteria-isolation locking connector according to claim 3, wherein the laparoscope locking apparatus (4) comprises laparoscopic locking buckles (41) and locking buckle spindles (42) provided through the laparoscopic locking buckles (41), two locking buckle spindles (42) and two fan-shaped bosses (114) are provided at intervals, and the two laparoscopic locking buckles (41) and the two locking buckle spindles (42) are symmetrically provided around the central axis of the laparoscope connector apparatus (1),
one end of the locking buckle spindle (42) is inserted into a first end face hole (13) of the laparoscope upper connector structure (11), and the other end of the locking buckle spindle (42) is inserted into the second end face hole (31) of the laparoscope connector upper cover (3).

7. The laparoscope bacteria-isolation locking connector according to claim 6, wherein the laparoscopic locking buckle (41) comprises an interconnected locking buckle body (411) and a first connection portion (412), the locking buckle body (411) is located on a side close to the laparoscope locking knob (2) and the first connection portion (412) is located on a side away from the laparoscope locking knob (2).

8. The laparoscope bacteria-isolation locking connector according to claim 7, wherein the laparoscopic locking buckle (41) further comprises a first protruding portion (413) provided at an end portion of the locking buckle body (411), the first protruding portion (413) is close to an inner wall of the laparoscope locking knob (2), and the inner wall of the laparoscope locking knob (2) is symmetrically provided with a second protruding portion (24) abutting against the first protruding portion (413) during the rotational locking of the laparoscope locking knob (2).

9. The laparoscope bacteria-isolation locking connector according to claim 2, wherein two semi-cylindrical protrusions (113) are symmetrically provided on the outer circumference of the upper cylindrical tube (111), the semi-cylindrical protrusions (113) are adapted to be connected with the semi-cylindrical clamping grooves (511) of the inner wall of the sliding table (5), and the inside of the laparoscope connector apparatus (1) is isolated from the sliding table (5) to form a sterile space.

10. A laparoscopic surgery robot, comprising a sliding table (5) and a laparoscope bacteria-isolation locking connector according to any of claims 1-9, and the laparoscope bacteria-isolation locking connector is fixedly connected to the sliding table (5).
